(19) Europäisches Patentamt
European Patent Office
Office européen des brevets

(11) Publication number: **0 169 176**
**A1**

# (12) EUROPEAN PATENT APPLICATION

(21) Application number: **85830156.7**

(51) Int. Cl.[4]: **C 07 C 101/30**

(22) Date of filing: **21.06.85**

(30) Priority: **04.07.84 IT 4850484**

(43) Date of publication of application: **22.01.86**
**Bulletin 86/4**

(84) Designated Contracting States: **AT BE CH DE FR GB LI LU NL SE**

(71) Applicant: **Sigma-Tau Industrie Farmaceutiche Riunite S.p.A., 47, Viale Shakespeare, I-00144 Rome (IT)**

(72) Inventor: **Cavazza, Claudio, 35, Via Marocco, I-00144 Rome (IT)**

(74) Representative: **Fassi, Aldo, Dr. et al, c/o Sigma-Tau Industrie Farmaceutiche Riunite S.p.A. Via Pontina, Km. 30, 400, I-00040 Pomezia (Roma) (IT)**

(54) **Tert-butyl gamma dimethylamino beta-hydroxy butyrate and process for its preparation.**

(57) Tert-butyl gamma dimethylamino beta-hydroxy butyrate (norcarnitine tert-butyl ester), a versatile intermediate useful for the preparation of carnitine and its derivatives which possess pharmaceutical activity, is prepared by reacting an alcoholic solution of the epoxy

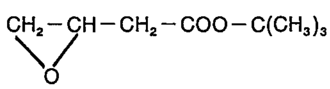

with an alcoholic solution of dimethylamine.

EP 0 169 176 A1

# Tert-butyl gamma dimethylamino beta-hydroxy butyrate and process for its preparation

The present invention relates to the novel chemical compound norcarnitine tert-butyl ester and to a process for its preparation.

Norcarnitine tert-butyl ester (tert-butyl gamma dimethylamino beta-hydroxy butyrate)

$$(CH_3)_2N-CH_2-\underset{OH}{\underset{|}{C}}H-CH_2-COO-C(CH_3)_3 \qquad (V)$$

is a useful intermediate for the preparation of carnitine and of carnitine derivatives such as, for example, acetyl carnitate of carnitine dichloride. The latter is a novel derivative of carnitine, whose chemico-physical characteristics and processes for preparation thereof are described in the Italian patent application 48503 A/84 filed on July 4, 1984 by the same applicant under the title "Ester of acetyl carnitine, processes for its preparation and pharmaceutical compositions containing it"

Norcarnitine tert-butyl ester is prepared according to the following reaction scheme:

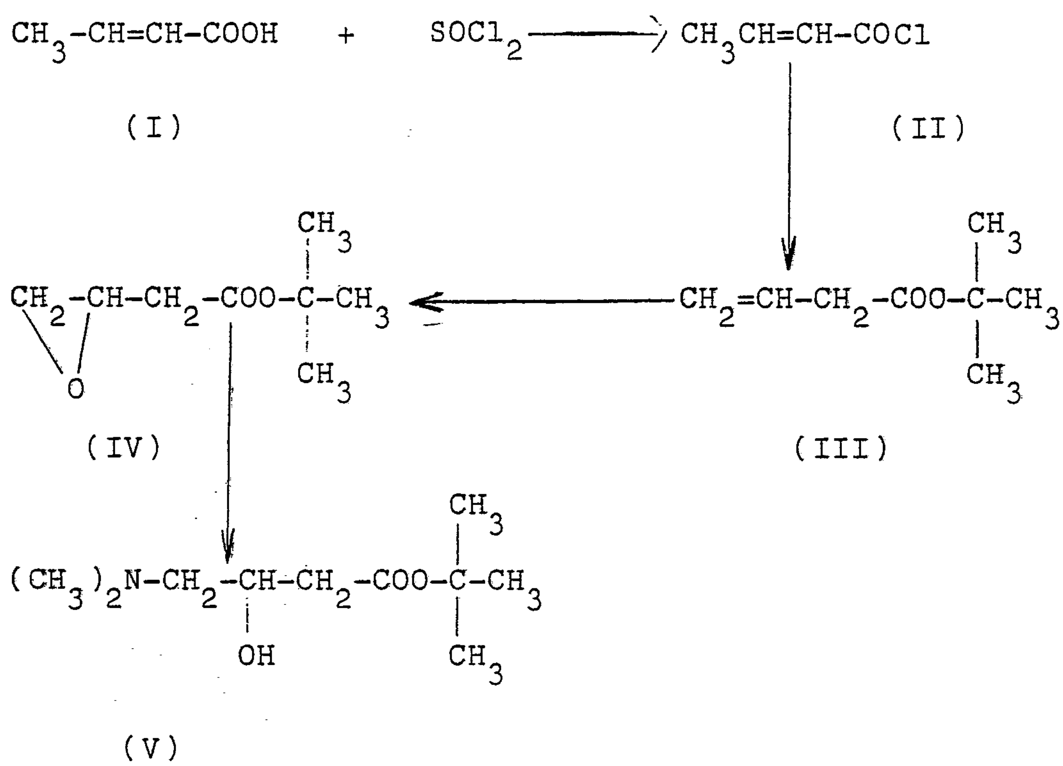

(I) (II) (IV) (III) (V)

The process of the present invention, however, is characterized by the sole step (IV)→(V), since the preceding steps (I)→(II), (II)→(III) and (III)→(IV) are already known and described in the literature.

In fact, the intermediate (II) may be synthesized as described in J.A.C.S. 58 , 2450, 1936, while the intermediates (III) and (IV) may be synthesized as described in J. Pharm. Science 64 , 1262, 1975.

More particularly, the process for the preparation of norcarnitine tert-butyl ester (V) according to the above-shown reaction scheme comprises the steps of:

(a) condensing crotonic acid (I) with thionyl chloride thereby obtaining crotyl chloride (II);

(b) reacting crotyl chloride (II) with tert-butanol, in the presence of an acid acceptor thereby obtaining tert-butyl venylacetate (III);

(c) epoxidizing tert-butyl vinylacetate (III) with m-chloroperbenzoic acid thereby obtaining the correspond ing epoxy (IV);

steps (a), (b) and (c) being carried out according to known processes, and it is characterized, according to the present invention, by the steps of:

(d) reacting a solution of the epoxy (IV) in lower alkanol with a solution of dimethylamine in lower alkanol, in a molar ratio (IV): dimethylamine comprised between 1:0.9 and 1:1.5, keeping the reaction mixture at about 5-30°C for about 3-8 hours in an inert gas atmosphere, thereby obtaining (V);

(e) isolating (V) from the reaction mixture of step (d) by concentrating the mixture under vacuum and treating the

residue with a water-immiscible solvent, extracting with a diluted acid solution and then alkalizing to a pH of about 12 and re-extracting with an organic solvent, anhydrizing the resulting organic phase and removing the organic solvent by distillation under reduced pressure.

Preferably, the lower alkanol of step (d) is methanol; the water-immiscible organic solvent of step (e) is ethyl ether or ethyl acetate.

As already mentioned, compound (V) is for example useful for the preparation of carnitine. At first sight it could seem that for preparing carnitine it is not necessary to start from compound (V), but that it suffices to react the epoxy (IV) directly with trimethylamine.

In contrast to what might seem obvious, the reaction does not prodeed in the desired direction (see J. Org. Chem. (1980) 45 , 2763-2766). It is therefore necessary to react the compound (V) with methyl iodide and to hydrolize the ester.

Example

(a) Preparation of crotyl chloride
(J.A.C.S. 58 , 2450, (1936) )

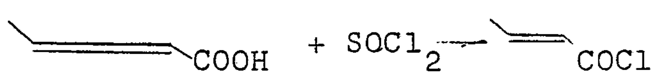

A mixture of 100 g of crotonic acid (1.163 mol) and 350 ml of $SOCl_2$ was refluxed in a 1 liter round-bottomed three-necked flask under mechanical stirring, until evolution of hydrochloric acid came to an end (about 6 hours). Distillation in a vigreux columm (h=30 cm) was carried out, thereby eliminating firstly the excess thionyl chloride (b.p. 75-80°C). The distillate obtained beteween 118 to 120°C was kept (pot temperature 124°C), thereby obtaining 80 g crotonyl chloride. Yield 65.8%.

(b) <u>Preparation of vinylacetate of tert-butyl</u>
(J.Pharm. Sci, <u>64</u> , 1262, (1975) )

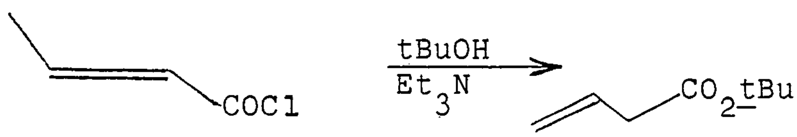


To a solution of distilled tert-butanol (107 ml, 1.15 mol) and of $Et_3N$ (106 ml, 0.765 mol) distilled over sodium in 270 ml of ethyl ether distilled over Na, there was added crotyl chloride (80 g, 0.727 mol) freshly distilled and dissolved in 40 ml ether.

In addition was effected dropwise and lasted about 1 hour, the temperature being kept at between 25 and 30°C (by means of a water-bath). At the end of the addition the mixture was kept under stirring for 1 hour at 25°C. Filtration was

carried out and the precipitate was washed with ethyl ether (2x50 ml). The filtrate was washed with cold 5% $NaHCO_3$ (100 ml), $H_2O$ (100 ml), cold 5% HCl (100 ml), $H_2O$ (100 ml), cold 5% $NaHCO_3$ (100 ml), $H_2O$ (100 ml). The organic phase was dried with $Na_2SO_4$.

The ether was distilled away from the orange coloured solution obtained and the residue was distilled under reduced pressure (18 mmHg). 62 g of pure ester were obtained as a pure, colourless liquid.

b.p. 39-40°C at 18 mmHg.

Yield 60%.

(c) Epoxidation of tert-butyl vinylacetate

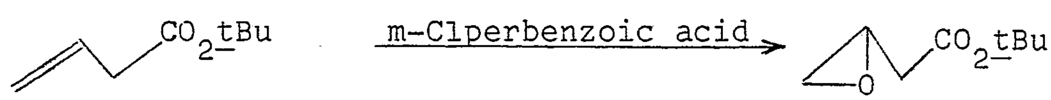


To a solution of 62 g (0.436 mol) of tert-butyl vinylacetate in 290 ml $CHCl_3$ cooled with ice and under mechanical stirring, there were slowly added 112.3 g (0.655 mol) of m-chloroperbenzoic acid previously washed with buffer at pH 7 in 1.8 lit. $CHCl_3$. The addition lasted about 3 hours, while the temperature was kept at between 0°C and 4°C. The resulting solution was kept at 4°C for 4 days. The precipitate

was then filtered and thoroughly washed with $CHCl_3$. The raw material, checked by gas chromatography (2,5% SE30 columm, Pressure $N_2$ = 1 atm, injector temperature = 220°C. Columm temperature 70°C for 7 minutes, then to 100°C at 10°C/min, then at 100°C for 5 minutes.), contained about 20% of starting material. The filtrate was kept under stirring with 2 litres of 10% $Na_2SO_3$ solution in $H_2O$ until the reaction on starch/iodide became negative (about 30 minutes). The organic phase was then washed with 250 ml of 5% $NaHCO_3$, 250 ml $H_2O$ and then was dried on $Na_2SO_4$. The $CHCl_3$ was distilled off with a vigreaux columm (h=30 cm) and the residue was then distilled off; 51.1g of product were obtained.

Yield 74%.

The epoxy on TLC silica gel, with hexane/AcOEt eluent 8:2, showed Rf 0.56 and developed with $I_2$.

(d) <u>Preparation of norcarnitine ter-butyl ester</u>

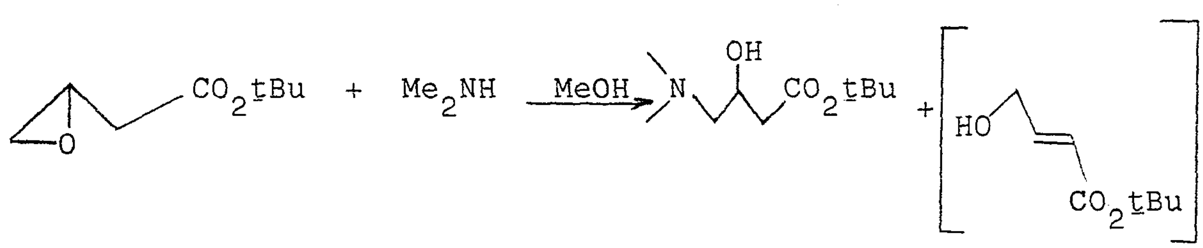


A solution of epoxy (1g; 6.3 mmol) in 3 ml anhydrous MeOH

was placed under nitrogen and cooled to 5-10°C. There were then added 680 mg of $Me_2NH$ in MeOH (5.9 mmol) and the temperature was allowed to rise to 25°C, while stirring was continued for 3 hours. The solvent was then evaporated under reduced pressure, the oil obtained was dissolved in 6 ml of ethyl ether and then extracted 3 times with 2 ml cold 5% HCl. The extract was brought to pH 12 with NaOH and the basic solution was extracted 3 times with 3 ml of ethyl acetate, which was then washed with 2 ml $H_2O$. The organic phase was anhydrized over $Na_2SO_4$ and the solvent was evaporated under reduced pressure. 700 mg norcarnitine tert-butyl ester were obtained. Yield 55%.

The product showed Rf 0.7 on TLC silica gel with $CHCl_3/MeOH/NH_3$ 80/20/2 and developed with $I_2$ it distilled at 100°C at 0.5 mmHg as a colourless liquid which quickly yellowed at room temperature.

C L A I M S

1. Norcarnitine tert-butyl ester

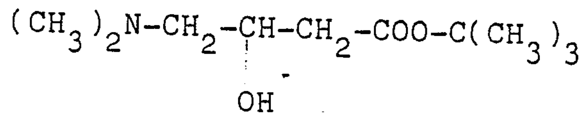

(V)

2. Process for the preparation of norcarnitine tert-butyl ester according to the reaction scheme:

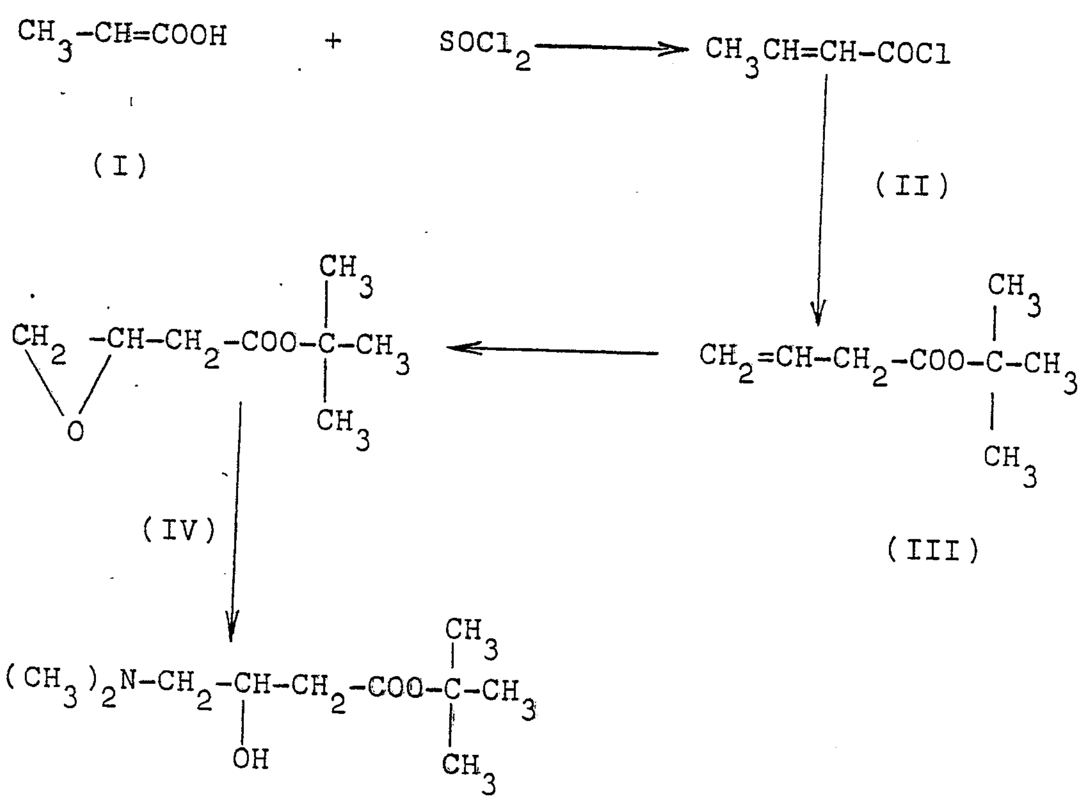


(V)

comprising the steps of:

(a) condensing crotonic acid (I) with thionyl chloride

thereby obtaining crotyl chloride (II);

(b) reacting crotyl chloride (II) with tert-butanol in the presence of an acid acceptor thereby obtaining tert-butyl vinylacetate (III);

(c) epoxidizing the tert-butyl vinylacetate (III) with m-chloroperbenzoic acid thereby obtaining the corresponding epoxy (IV);

the steps (a), (b) and (c) being carried out according to known processes, characterized by the steps of:

(d) reacting a solution of the epoxy (IV) in lower alkanol with a solution of dimethylamine in lower alkanol, with a molar ratio (IV): dimethylamine comprised between 1:0.9 and 1:1.5, keeping the reaction mixture at about 5-30°C for about 3-8 hours in an inert gas atmosphere, thereby obtaining (V);

(e) isolating (V) from the reaction mixture of step (d) by concentrating the mixture under vacuum and treating the residue with a water-immiscible solvent, extracting with a diluted acid solution and then alkalizing to a pH of about 12 and re-extracting with an organic solvent, anhydrizing the resulting organic phase and removing the organic solvent by distillation under reduced pressure.

3. Process according to claim 2, characterized in that in step (d) the lower alkanol is methanol.

4. Process according to claims 2 or 3, characterized in that in step (e) the water-immiscible organic solvent is selected from ethyl ether and ethyl acetate.

Single claim for Austria (under the provision of art. 167(2)(a) EPC).

## CLAIM

Process for the preparation of norcarnitine tert-butyl ester having the formula

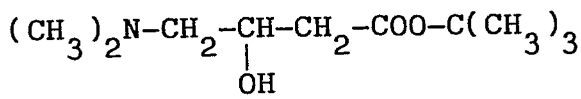

(V)

according to the reaction scheme:

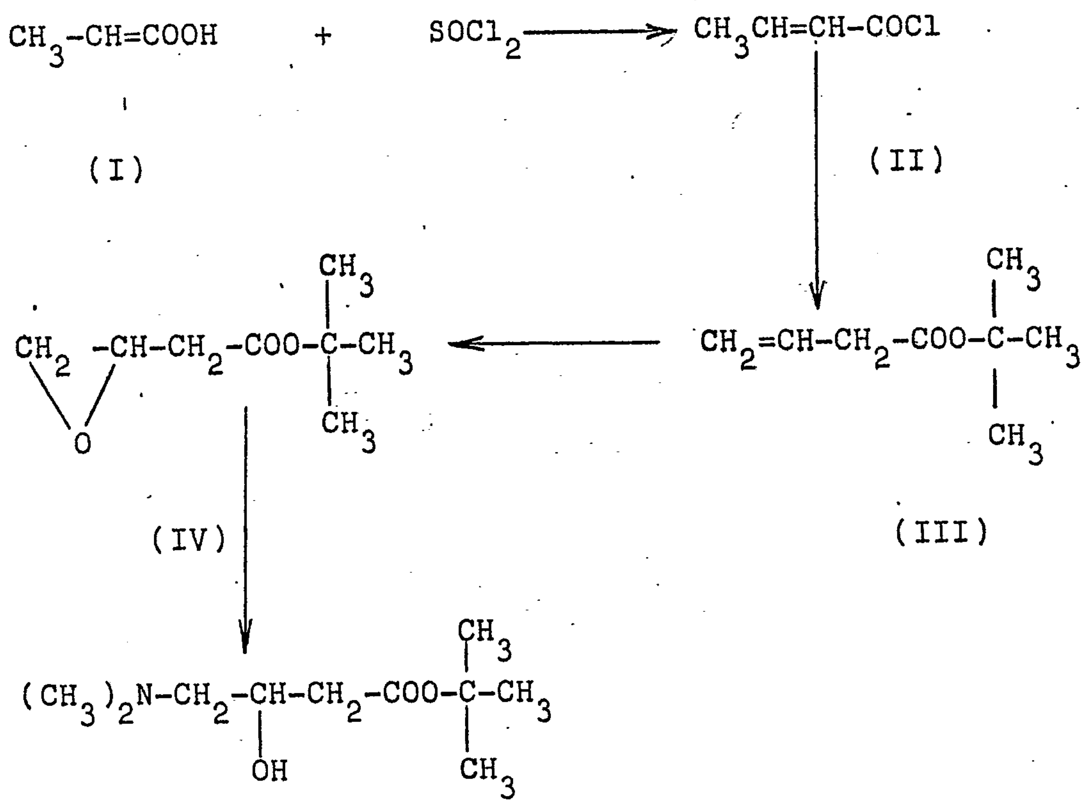


(V)

comprising the steps of:

(a) condensing crotonic acid (I) with thionyl chloride thereby obtaining crotyl chloride (II);

(b) reacting crotyl chloride (II) with tert-butanol in the presence of an acid acceptor thereby obtaining tert-butyl vinylacetate (III);

(c) epoxidizing the tert-butyl vinylacetate (III) with m-chloroperbenzoic acid thereby obtaining the corresponding epoxy (IV);

the steps (a), (b) and (c) being carried out according to known processes, characterized by the steps of:

(d) reacting a solution of the epoxy (IV) in lower alkanol with a solution of dimethylamine in lower alkanol, with a molar ratio (IV): dimethylamine comprised between 1:0.9 and 1:1.5, keeping the reaction mixture at about 5-30°C for about 3-8 hours in an inert gas atmosphere, thereby obtaining (V);

(e) isolating (V) from the reaction mixture of step (d) by concentrating the mixture under vacuum and treating the residue with a water-immiscible solvent, extracting with a diluted acid solution and then alkalizing to a pH of about 12 and re-extracting with an organic solvent, anhydrizing the resulting organic phase and removing the organic solvent by distillation under reduced pressure.

European Patent Office

**EUROPEAN SEARCH REPORT**

0169176

Application number

EP 85 83 0156

**DOCUMENTS CONSIDERED TO BE RELEVANT**

| Category | Citation of document with indication, where appropriate, of relevant passages | Relevant to claim | CLASSIFICATION OF THE APPLICATION (Int Cl 4) |
|---|---|---|---|
| Y | THE JOURNAL OF ORGANIC CHEMISTRY, vol. 45, no. 14, 4th July 1980, pages 2763-2766, American Chemical Society, US; C.R. DEGENHARDT: "Synthesis of carnitine homologues. Reactions of tertiary amines with epoxy esters" * Page 2763 * | 1-4 | C 07 C 101/30 |
| Y | CHEMICAL ABSTRACTS, vol. 59, no. 3, 5th August 1963, column 2654c-d, Columbus, Ohio, US; & JP - A - 62 5172, JP - A - 62 5173, JP - A - 62 5174 (FUJISAWA PHARMACEUTICAL CO., LTD.) 19-06-1962 | 1-4 | |
| | | | TECHNICAL FIELDS SEARCHED (Int. Cl.4) |
| D,Y | JOURNAL OF PHARMACEUTICAL SCIENCES, vol. 64, no. 7, July 1975, pages 1262-1264; S.G. BOOTS et al.: "New synthesis of (RS)-carnitine chloride" * Page 1263 * | 1-4 | C 07 C 101/00 |

The present search report has been drawn up for all claims

| Place of search | Date of completion of the search | Examiner |
|---|---|---|
| THE HAGUE | 10-10-1985 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82